(19) Europäisches Patentamt
European Patent Office
Office européen des brevets

(11) Publication number: **0 149 289**
**B1**

(12) # EUROPEAN PATENT SPECIFICATION

(45) Date of publication of patent specification: **04.01.89**

(51) Int. Cl.[4]: **C 07 D 307/93** // C07C62/18, C07C69/14

(21) Application number: **84300215.5**

(22) Date of filing: **13.01.84**

(54) **Process for the manufacture of an optically active lactone of a cyclopropane-carboxylic acid.**

(43) Date of publication of application:
**24.07.85 Bulletin 85/30**

(45) Publication of the grant of the patent:
**04.01.89 Bulletin 89/01**

(84) Designated Contracting States:
**CH DE FR GB IT LI NL**

(56) References cited:
**EP-A-0 031 041**
**US-A-4 219 562**

**CHEMICAL ABSTRACTS, vol. 101, no. 5, 30th July 1984, abstract no. 38671m, Columbus, Ohio, US; R.H. NAIK et al.: "Syntheis of methyl 1R-(-)-cis- and (+-)-cis-2,2-dimethyl-3-(2-oxopropyl)cyclopropanecarboxylates from (+)-3-carene" & INDIAN J. CHEM., SECT: B 1983, 22B(12), 1209-12**

**THE MERCK INDEX, 9th edition, 1976, page ONR-5, "Baeyer-Villiger reactions", Merck & co., Inc., Rahway, N.J., US;**

(73) Proprietor: **IEL Limited**
**ICI House 34, Chowringhee Road**
**Calcutta 700071 West Bengal (IN)**

(72) Inventor: **Mandal, Arun Kanti**
**Alchemie Research Centre PVT Ltd. Belapur Road**
**Thane 400601 Maharashtra (IN)**
Inventor: **Bhandari, Shailendra Ratanchand**
**Alchemie Research Centre PVT Ltd. Belapur Road**
**Thane 400601 Maharashtra (IN)**
Inventor: **Mahajan, Satish Wasudeo**
**Alchemie Research Centre PVT Ltd. Belapur Road**
**Thane 400601 Maharashtra (IN)**

(74) Representative: **Collier, Jeremy Austin Grey et al**
**J.A.Kemp & Co. 14, South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 149 289 B1**

## Description

The present invention relates to the manufacture of the optically active γ-lactone of (1R, cis)-2,2-dimethyl-3-hydroxymethyl-cyclopropane-1-carboxylic acid (hereinafter referred to as optically active (1R, cis)-γ-lactone or optically active γ-lactone) and more particularly to the synthesis of an optically active γ-lactone from optically active car-3-en-5-one.

The optically inactive γ-lactone or *cis*-2,2-dimethyl-3-hydroxy-methyl-cyclopropane-carboxylic acid, having the formula

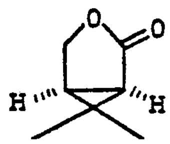

(I)

is a known compound which may be manufactured by a multi-step process. It is a well known intermediate for the manufacture of optically inactive methyl *cis*-2,2-dimethyl-3-formyl-cyclopropane-carboxylate of the formula II below, itself a valuable intermediate for the synthesis of optically inactive *cis*-chrysanthemic acid of the formula III below, see A. Krief *et al. Tet. Lett* 3915 (1976), and the pyrethroid acid of formula IV below, see J. Martel *et al*, DE—A—2827627 (1979), Roussel-Uclaf.

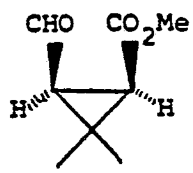

(II)

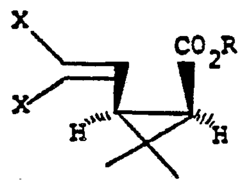

(III)
($X=CH_3$ or R=H)

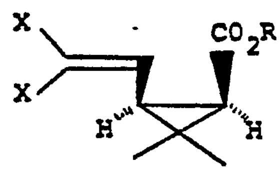

(IV)
(X=Cl or Br and R=H)

The aforesaid optically active γ-lactone is also convenient starting material for the manufacture of optically active (1R, cis)-pyrethroid acid of formula IV which can be converted in an to an optically active (1R, cis)-synthetic pyrethroid having the formula III above, where X = Cl and

R = 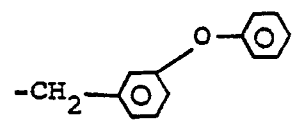 or X = Cl or Br and R = 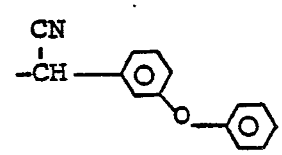

The above optically active (1R, *cis*)-synthetic pyrethroids possess more insecticidal activity than the corresponding optically inactive compounds.

EP—A—31041 relates to insecticides and acaricides which are 3-phenoxy-benzyl or α-cyano-3-phenoxy-benzyl esters of 2,2-dimethylcyclopropane carboxylic acids substituted in position 3 by a dienyl polyhalogenated chain. In particular, a possible process for making these compounds includes the step of hydrolysing AcO—$CH_2$

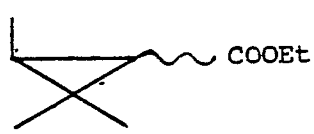 to give a mixture of

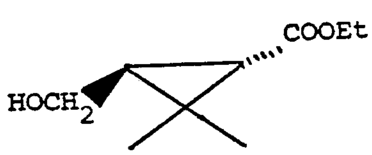 and 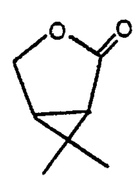

The present invention provides a process for producing the said optically active γ-lactone which is short and commercially convenient. The new process avoids the use of a hazardous ozonolysis reaction as in prior art processes for the manufacture from (+)-3-carene of various intermediates useful for the manufacture of pyrethroid insecticides.

The process of the present invention for the manufacture of optically active γ-lactone of (1R, cis)-2,2-dimethyl-3-hydroxymethyl-cyclopropane-1-carboxylic acid comprises oxidizing optically active (−)car-3-en-5-one of formula:

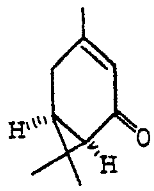

(V)

to form optically active (−)-(1R, cis)-2,2-dimethyl-3-(2-oxopropyl)-cyclopropane-1-carboxylic acid of formula:

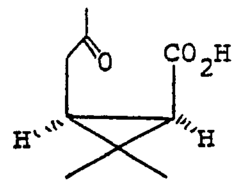

(VI)

oxidising the said compound of formula (VI) to form (1R, cis)-2,2-dimethyl-3-(acetoxymethyl)-cyclopropane-1-carboxylic acid of formula:

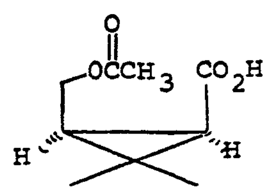

(VII)

and hydrolysing the compound of formula (VII) to form the optically active γ-lactone.

The compound of formula (V) may be obtained by known methods, such as oxidation of (+)-3-carene formula of the formula

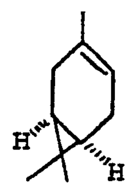

(VIII)

with air in the presence of a catalyst as described in U.S. Patent 4,933,244.

The invention, thus, also provides a short process for the manufacture from (+)-3-carene of the optically active compound of formula VI which in the prior art has been manufactured by multi-step processes, see Matsui *et al*, Agric. Biol. Chem. Jap. *21*, 784 (1965); R. B. Mitra *et al*, Ind. J. Chem., *208* 436 (1981); (+)-(VI), W. Cocker *et al*, J. C. S. Perkin I, 332 (1975).

The first oxidation reaction to make the intermediate compound of formula (VI) may be conducted in the liquid phase, at ambient temperature, using known oxidants, such as potassium permanganate, mixture of potassium permanganate/chromic acid, potassium permanganate-potassium dichloromate (or sodium dichlromate), potassium permanganate-sodium periodate and the like, in solvents such as water, acetone-acetic acid-water, or acetic acid-water mixture.

The compound of formula (VI) is oxidised to the carboxylate derivative, preferably the acetate derivative of the formula (VII) with known oxidants such as hydrogen peroxide, per-acids such as *m*-chloroperbenzoic acid, perbenzoic acid, perphthalic acid, peracetic acid and the like in suitable chlorinated solvents such as methylene chloride, chloroform or acetic acid at room temperature.

Hydrolysis of the carboxylate of the formula (VII) to the said optically active γ-lactone of formula I is conducted preferably in the same reaction zone or vessel as the said oxidation with per-acids, preferably under acidic condition using aqueous inorganic acids such as sulphuric acid, hydrochloric acid or the like, in polar solvents such as methanol, ethanol, dioxane, tetrahydrofuran, acetic acid or the like.

The invention is illustrated by the following Examples. The identity of the products, including intermediates, and purity was confirmed by spectroscopic and GLC analysis as necessary.

Example 1

(−)-(1R, cis)-2,2-dimethyl-3-(2-oxopropyl)-cyclopropane-1-carboxylic acid

(−)-car-3-en-5-one (15 g, 0.1 mole) was dissolved in (1:1) acetic acid-water (250 ml). To the vigorously stirred solution, solid potassium permanganate (79 g, 0.5 mole) was added portion-wise at ambient temperature. The reaction mixture was further stirred for an hour. Sulphur dioxide was passed through the reaction mixture and the residue was extracted with 3 × 150 ml of chloroform. The combined chloroform layer was washed with saturated brine solution (100 ml), dried over anhydrous magnesium sulphate and filtered. The solvent was stripped off under vacuum to yield 12.5 g of the product (75% yield) as a yellow viscous oil $[\alpha]_D^{25} = -30°$ ($C = 0.5$ $CHCl_3$).

Example 2

γ-lactone of (−)-(1R, cis)-dimethyl-3-hydroxymethylcyclopropane-1-carboxylic acid

To a solution of the product from Example 1 (5.1 g, 0.03 mole) in dichloromethane (10 ml) was added with stirring at ambient temperature a solution of 85% *m*-chloroperbenzoic acid (7.2 g, 0.036 mole) in dichloromethane (20 ml). The reaction mixture was stirred for 72 h. The precipitated *m*-chlorobenzoic acid was filtered off. The filtrate, after removal of solvent, was treated with methanol (10 ml) and 10% hydrochloric acid (10 ml) and stirred overnight. The reaction mixture was extracted with dichloromethane (50 ml). The organic layer was thoroughly washed with 10% aqueous sodium carbonate solution, dried over anhydrous sodium sulphate and filtered. Removal of solvent yielded the γ-lactone (3.0 g, 85% yield) as a light yellow oil, $[\alpha]_D^{25} = -22.5°$ ($C = 0.6$, $CHCl_3$).

**Claims**

1. A process for the manufacture of optically active γ-lactone of (1R, cis)-2,2-dimethyl-3-hydroxymethylcyclopropane-1-carboxylic acid which comprises oxidising optically active (−)car-3-en-5-one of formula:

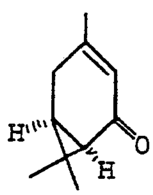

to form optically active (−)-(1R, cis)-2,2-dimethyl-3-(2-oxopropyl)-cyclopropane-1-carboxylic acid of formula:

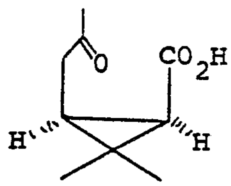

oxidising the said acid to form (1R, cis)-2,2-dimethyl-3-(acetoxymethyl)-cyclopropane-1-carboxylic acid of formula:

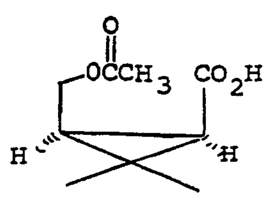

and hydrolysing the said compound to form the optically active γ-lactone .

2. A process as claimed in claim 1 wherein the said starting material is obtained by the oxidation of (+)-3-carene of the formula:

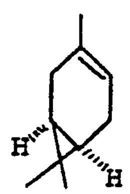

3. A process as claimed in claim 1 or 2 wherein the oxidation of the carenone compound is conducted in the liquid phase, at ambient temperature using as oxidant potassium permanganate, a mixture of potassium permanganate and chromic acid, potassium permanganate and potassium dichromate or sodium dichromate, or potassium permanganate and sodium periodate, in an inert solvent.

4. A process as claimed in claim 1, 2 or 3 wherein the oxidation of the cyclopropane-carboxylic acid is carried out with hydrogen peroxide or a per-carboxylic acid in an inert organic solvent at ambient temperature.

5. A process as claimed in any of claims 1 to 4 wherein the hydrolysis of the intermediate compound to form the lactone is conducted in the same reaction zone as the preceding oxidation without isolating the said intermediate.

6. A process as claimed in claim 5 wherein the said hydrolysis is effected in acidic conditions in a polar solvent.

**Patentansprüche**

1. Verfahren zur Herstellung eines optisch aktiven γ-lactone einer (1R, cis)-2,2-Dimethyl-3-hydroxymethylcyclopropan-1-Carbonsäure durch Oxidation von optisch aktivem (−)Car-3-en-5-on der Formel:

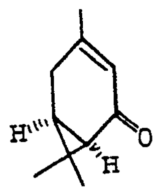

zwecks Bildung optisch aktiver (−)-(1R, cis)-2,2-Dimethyl-3-(2-oxopropyl)-cyclopropan-1-carbonsäure der Formel:

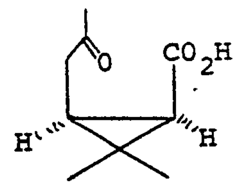

druch Oxidation dieser Säure zwecks Bildung von (1R, cis)-2,2-Dimethyl-3-(acetoxymethyl)-cyclopropan-1-carbonsäure der Formel:

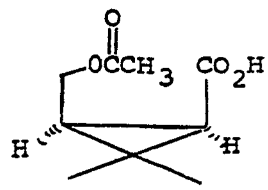

und durch Hydrolyse dieser Verbindung zwecks Bildung des optisch aktiven γ-Lactons.

2. Verfahren nach Anspruch 1, wobei das erwähnte Ausgangsmaterial durch Oxidation von (+)-3-Caren der Formel:

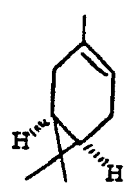

erhalten wird.

3. Verfahren nach Anspruch 1 oder 2, wobei die Oxidation der Carenonverbindung in flüssiger Phase und bei Raumtemperatur unter Verwendung von Kaliumpermanganat, einer Mischung von Kaliumpermanganat und Chromsäure, Kaliumpermanganat und Kaliumdichromat oder Natriumdichromat oder Kaliumpermanganat und Natriumperjodat als Oxidationsmittel in einem inerten Lösungsmittel durchgeführt wird.

4. Verfahren nach Anspruch 1, 2 oder 3, wobei die Oxidation der Cyclopropancarbonsäure mit Wasserstoffperoxid oder einer Percarbonsäure in einem inerten organischen Lösungsmittel bei Raumtemperatur durchgeführt wird.

5. Verfahren nach einem der Ansprüche 1 bis 4, wobei die Hydrolyse des Zwischenprodukts zwecks Bildung des Lactons in derselben Reaktionszone durchgeführt wird wie die vorausgehende Oxidation, ohne daß das erwähnte Zwischenprodukt isoliert wird.

6. Verfahren nach Anspruch 5, wobei die Hydrolyse unter sauren Bedingungen in einem polaren Lösungsmittel durchgeführ wird.

**Revendications**

1. Un procédé pour la préparation de la gamma-lactone, possédant l'activé optique, de l'acide (1R, cis)-2,2-diméthyl-3-hydroxyméthyl-cycloprane-1-carboxylique qui consiste à oxyder la (−)-cara-3-ène-5-one possédant l'activité optique, de formule

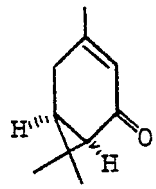

avec formation de l'acide (−)-(1R, cis)-2,2-diméthyl-3-(2-oxopropyl)cyclopropane-1-carboxylique, possédant l'activité optique, de formule

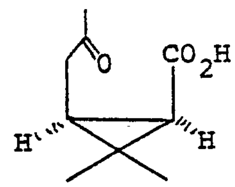

qu'on oxyde avec formation de l'acide (1R, cis)-2,2-diméthyl-3-(acétoxyméthyl)-cyclopropane-1-carboxylique de formule

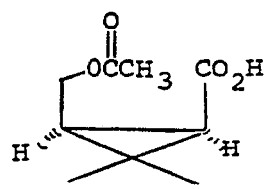

dont l'hydrolyse donne la gamma-lactone possédant l'activité optique.

2. Un procédé selon la revendication 1, dans lequel le produit de départ utilisé est obtenu par oxydation du (+)-3-carène de formule

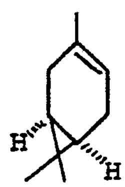

3. Un procédé selon la revendication 1 ou 2, dans lequel l'oxydation de carénone est effectuée en phase liquide, à température ambiante, à l'aide d'un oxydant consistant en le permanganate de potassium, un mélange de permanganate de pottasium et d'acide chromique, un mélange de permanganate de potassium et de bichromate de potassium ou de bichromate de sodium ou un mélange de permanganate de potassium et du periodate de sodium dans un solvant inerte.

4. Un procédé selon la revendication 1, 2 ou 3, dans lequel l'oxydation de l'acide cyclopropane-carboxylique est effectuée à l'aide de peroxyde d'hydrogène ou d'un acide percarboxylique dans un solvant organique inerte à température ambiante.

5. Un procédé selon l'une quelconque des revendications 1 à 4, dans lequel l'hydrolyse du composé intermédiaire pour formation de la lactone est réalisée dans la même zone de rèaction que l'oxydation précédente, sans isolement dudit composé intermédiaire.

6. Un procédé selon la revendication 5, dans lequel l'hydrolyse est effectuée en milieu acide dans un solvant polaire.